# EUROPEAN PATENT APPLICATION

(11) **EP 4 644 406 A1**
(43) Date of publication of application: **05.11.2025**
(21) Application number: 23912093.4
(22) Date of filing: 26.12.2023
(51) Int. Cl.: C07K 5/08, A61K 51/08, A61K 51/10, C07F 5/00, C07K 16/18, C07K 16/28

(54) **RADIOLABELED MEDICINE**

(30) Priority: 27.12.2022 JP 2022210738
(71) Applicant: National University Corporation Chiba University, Chiba-shi, Chiba 263-8522 (JP)
(72) Inventor: UEHARA Tomoya, Chiba-shi, Chiba 260-8675 (JP); SUZUKI Hiroyuki, Chiba-shi, Chiba 260-8675 (JP)
(74) Representative: Hoffmann Eitle
(86) International application number: PCT/JP2023/046536
(87) International publication number: WO 2024/143314

(57) **Abstract**

It is an object of the present invention to provide a novel compound for use in preparation of a radiolabeled pharmaceutical capable of reducing accumulation in the kidney. As a solution, the present invention provides the following: A compound represented by the following formula (1) or a pharmaceutically acceptable salt thereof: 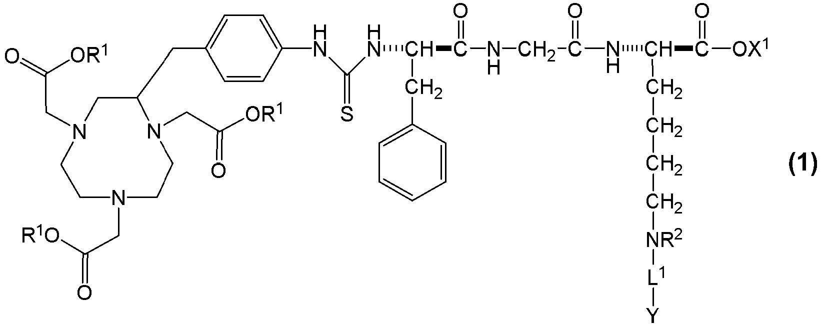 wherein R¹, R², L¹, Y, and X¹ are as described in the specification.

## Description

### Technical Field

The present invention relates to a novel compound, a composition for preparing a radiolabeled pharmaceutical, a radiolabeled pharmaceutical composition, a method of producing a novel compound, and the like.

### Background Art

Radiolabeled pharmaceuticals are used in radiographic diagnostic imaging, such as Single Photon Emission Computed Tomography (hereinafter, also referred to simply as "SPECT") and Positron Emission Tomography (hereinafter, also referred to simply as "PET"). Various radioactive nuclides are used in these types of radiographic diagnostic imaging, and among the radioactive nuclides, radioactive gallium can be used with a device for either type, as gallium-67 (⁶⁷Ga) can be used for SPECT and gallium-68 (⁶⁸Ga) can be used for PET. In particular, ⁶⁸Ga has been attracting attention in recent years, because it can be eluted from a generator using germanium-68.

Since ⁶⁸Ga has a short half-life of 68 minutes, a peptide with a small molecular weight, such as a miniaturized antibody with a rapid blood clearance, is used as its labeling base. However, when a radiolabeled low-molecular peptide is administered to an organism, in addition to specific accumulation in the target tissue, non-specific accumulation in the kidney is observed. In such a case, it becomes difficult to image the target tissue near the kidney. The accumulation of the radioactivity in the kidney (hereinafter, also referred to as "kidney accumulation") occurs because the RI-labeled low-molecular peptide after being taken into the kidney is delivered to the lysosome and metabolized, and its radioactive metabolite then produced remains in the kidney.

PTL 1 discloses NOTA-Met-Val-Lys(Mal) as a compound which achieves a radiolabeled drug capable of reducing the accumulation in the kidney from the early stage after the administration.

### Citation List

### Patent Literature

PTL 1: International Publication No. WO 2017/150549

### Summary of Invention

### Technical Problem

Since ⁶⁸Ga has the short half-life of 68 minutes, it is desirable to reduce the accumulation in the kidney from the early stage after the administration.

When NOTA-Met-Val-Lys(Mal) described in PTL 1 is used, the reduction of the accumulation in the kidney can be achieved. However, the present inventors found that accumulation in the kidney was observed, although only slightly, from ten minutes to one hour after the administration.

The present invention relates to a novel compound for use in preparation of a radiolabeled pharmaceutical capable of reducing more such accumulation in the kidney, and the like.

### Solution to Problem

The present invention relates to the following inventions:
[1] A compound represented by the following formula (1) or a pharmaceutically acceptable salt thereof: 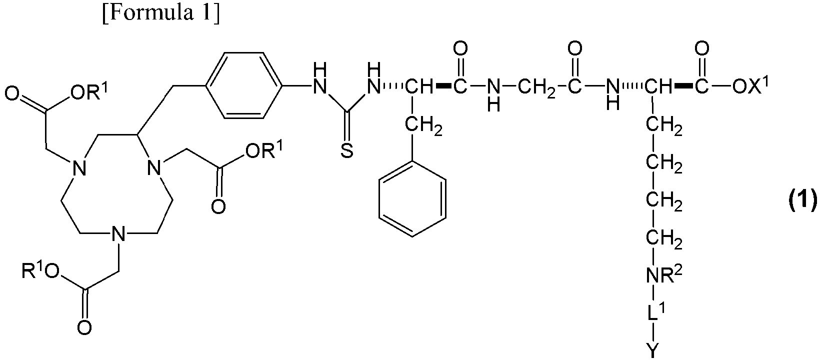 wherein
   R¹s are each independently a hydrogen atom or a hydrocarbon group having 1 to 8 carbon atoms,
   R² is a hydrogen atom or an organic group having 1 to 8 carbon atoms,
   L¹ is a single bond or a linker group,
   Y is a monovalent group P derived from a protein or a peptide, a hydrogen atom, or a target molecule recognition element,
   X¹ is a hydrogen atom or a pharmaceutically acceptable cation, and
   R² and L¹ are capable of binding to each other to form a nitrogen-containing heterocycle having 3 to 10 carbon atoms together with the adjacent nitrogen atom.
[2] The compound or a pharmaceutically acceptable salt thereof according to [1], wherein Y is a monovalent group P derived from a protein or a peptide.
[3] A composition for preparing a radiolabeled pharmaceutical, containing the compound or a pharmaceutically acceptable salt thereof according to [1] or [2].
[4] A compound represented by the following formula (2) or a pharmaceutically acceptable salt thereof: 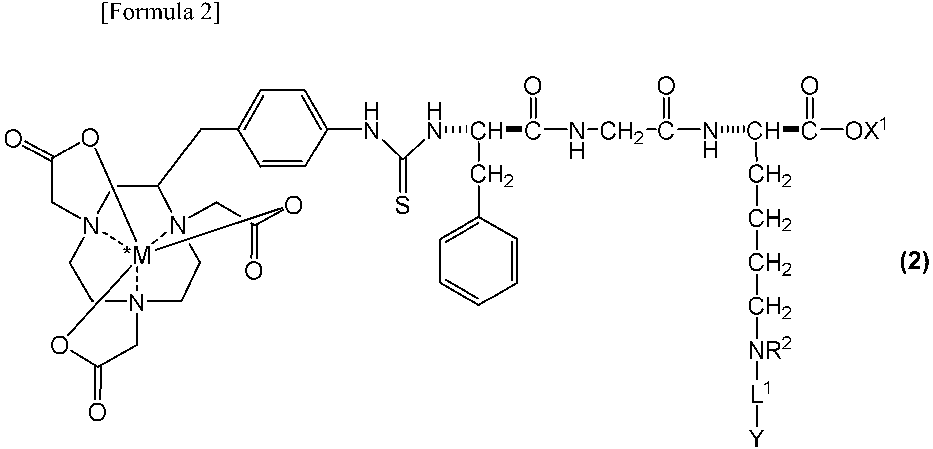 wherein
   *M is ⁶⁷Ga, ⁶⁸Ga, ¹⁸F-Al, ⁶⁴Cu, or ⁶⁷Cu,
   R² is a hydrogen atom or an organic group having 1 to 8 carbon atoms,
   L¹ is a single bond or a linker group,
   Y is a monovalent group P derived from a protein or a peptide, a hydrogen atom, or a target molecule recognition element,
   X¹ is a hydrogen atom or a pharmaceutically acceptable cation, and
   R² and L¹ are capable of binding to each other to form a nitrogen-containing heterocycle having 3 to 10 carbon atoms together with the adjacent nitrogen atom.
[5] The compound or a pharmaceutically acceptable salt thereof according to [4], wherein Y is a monovalent group P derived from a protein or a peptide.
[6] A radiolabeled pharmaceutical composition containing the compound or a pharmaceutically acceptable salt thereof according to [4] or [5].
[7] A method of producing the compound or a pharmaceutically acceptable salt thereof according to [1] or [2], the method being one of the method (I) including the following steps (a1) to (a2) and the method (II) including the following steps (b1) to (b2):
   {method (I)}
      (a1) a step of reacting an isothiocyanate group of a compound represented by the following formula (i) with an N-terminus of a phenylalanine residue of a compound represented by the following formula (ii);
      (a2) a step of reacting a linker group L^{1a} introduced into an amino group in a side chain of a lysine residue of the compound obtained in the step (a1), or the amino group, with a linker group L^{1b} or a molecular end of P of a compound represented by the following formula (iii),
   {method (II)}
      (b1) a step of reacting the linker group L^{1b} or the molecular end of P of a compound represented by the following formula (iii) with the linker group L^{1a} introduced into the amino group in the side chain of the lysine residue of the compound represented by the following formula (ii), or a step reacting the molecular end of P of a compound represented by the following formula (iii) with the amino group in the side chain of the lysine residue of the compound represented by the following formula (ii);
      (b2) a step of reacting the amino group of the phenylalanine residue at the N-terminus of the compound obtained in the step (b1) with the isothiocyanate group of the compound represented by the following formula (i): 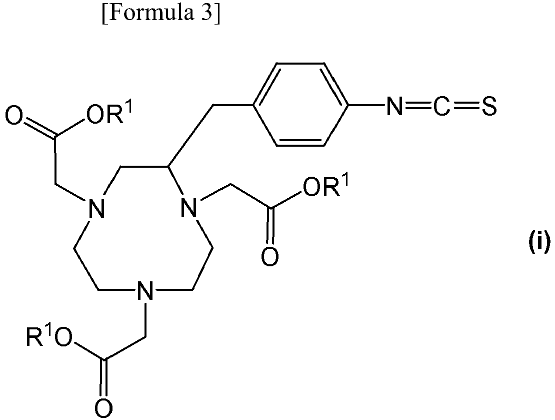
      [0011] wherein R¹s are each independently a hydrogen atom or a hydrocarbon group having 1 to 8 carbon atoms, 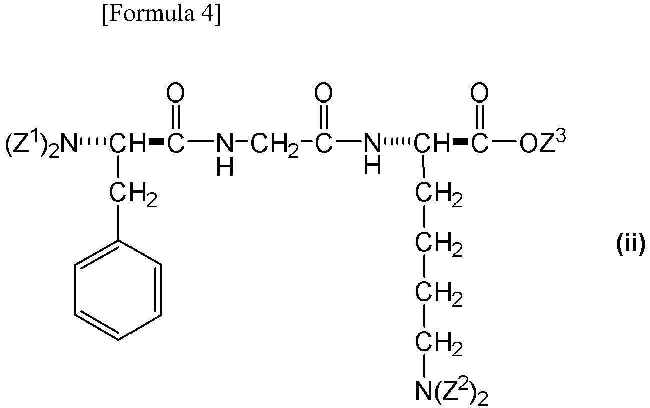
      [0013] wherein Z¹ and Z² are each independently a hydrogen atom or a protecting group of an amino group, and
      Z³ is a hydrogen atom, a protecting group of a carboxy group, or a bond with a solid phase,
         [Formula 5]

         (L^{1b})ₘ-P **(iii)**
      wherein m is 0 or 1,
      when m is 0, P is a protein or a peptide, and
      when m is 1, L^{1b} is a linker group and P is a monovalent group derived from a protein or a peptide.
[8] A method of producing the compound or a pharmaceutically acceptable salt thereof according to [4] or [5], including a step of reacting the compound or a pharmaceutically acceptable salt thereof according to [1] or [2] with a salt of ⁶⁷Ga, ⁶⁸Ga, ¹⁸F-Al, ⁶⁴Cu, or ⁶⁷Cu.

### Advantageous Effects of Invention

According to the present invention, the novel compound for use in preparation of the radiolabeled pharmaceutical capable of reducing more the accumulation in the kidney, and the like can be provided.

### Brief Description of Drawing

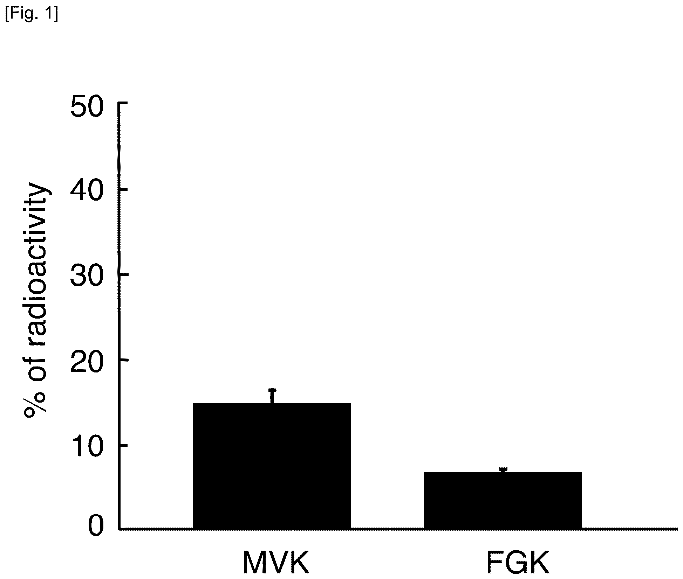

[Fig. 1] Fig. 1 illustrates results of experiments in which ⁶⁷Ga-labeled NOTA-Phe-Gly-Lys(Bzo) or ⁶⁷Ga-labeled NOTA-Met-Val-Lys(Bzo) is incubated with BBMVs.

### Description of Embodiments

### [Compound or the Like]

### <Compound (1) or the Like>

Hereinafter, a compound represented by the following formula (1) or a pharmaceutically acceptable salt thereof (hereinafter, also referred to simply as "compound (1) or the like") according to the present invention will be described in detail: 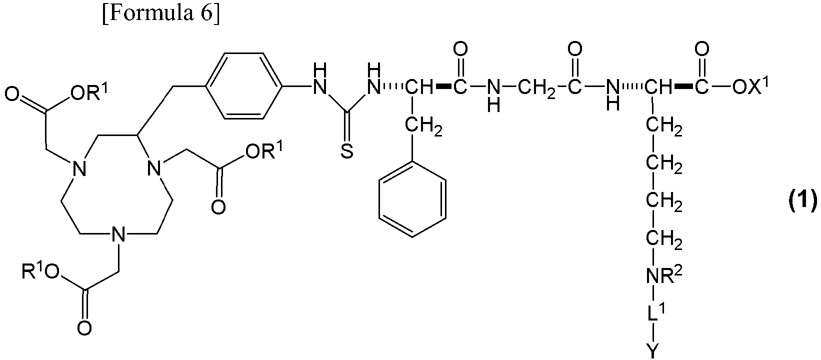 wherein
R¹s are each independently a hydrogen atom or a hydrocarbon group having 1 to 8 carbon atoms,
R² is a hydrogen atom or an organic group having 1 to 8 carbon atoms,
L¹ is a single bond or a linker group,
Y is a monovalent group P derived from a protein or a peptide, a hydrogen atom, or a target molecule recognition element,
X¹ is a hydrogen atom or a pharmaceutically acceptable cation, and
R² and L¹ are capable of binding to each other to form a nitrogen-containing heterocycle having 3 to 10 carbon atoms together with the adjacent nitrogen atom.

R¹s above are each independently a hydrogen atom or a hydrocarbon group having 1 to 8 carbon atoms.

The hydrocarbon group of R¹ is not particularly limited, and is preferably a hydrocarbon group used as a protecting group of a carboxy group, including, for example, a methyl group, an ethyl group, a t-butyl group, and a benzyl group.

Among these R¹s, a hydrogen atom is preferable.

R² above is a hydrogen atom or an organic group having 1 to 8 carbon atoms.

Further, L¹ above is a single bond or a linker group.

R² and L¹ are capable of binding to each other to form a nitrogen-containing heterocycle having 3 to 10 carbon atoms together with the adjacent nitrogen atom. In other words, the nitrogen-containing heterocycle may or may not be formed. Examples of the nitrogen-containing heterocycle having 3 to 10 carbon atoms which is formed by R² and L¹ bound to each other together with the adjacent nitrogen atom include a maleimide group.

The organic group having 1 to 8 carbon atoms of R² above is an organic group preferably having 1 to 5 carbon atoms, and more preferably having 1 to 3 carbon atoms. Examples of the organic group include, for example, an alkyl group, an alkoxy group, and an acyl group.

Y is a monovalent group P derived from a protein or a peptide, a hydrogen atom, or a target molecule recognition element. In one embodiment, Y is a hydrogen atom or a monovalent group P derived from a protein or a peptide.

The protein or the peptide of "a monovalent group P derived from a protein or a peptide" is not particularly limited, and a protein or a peptide capable of binding to a target molecule, such as a protein, present in an organism is preferably used. In detail, examples of such a protein or a peptide include proteins or peptides which function as a ligand capable of binding to a protein which is highly expressed in tissue with inflammation, tumor cell infiltration, or the like, a protein which is specifically expressed in a tumor cell, or the like.

Examples of such a protein or a peptide include antibodies and antigen-binding region fragments thereof.

Examples of the antigen-binding region fragment of an antibody include, for example, a Fab fragment (hereinafter, also referred to simply as "Fab"), an F(ab')₂ fragment, an F(ab)₂ fragment, and a variable region fragment (hereinafter, also referred to as "Fv fragment"). The term "Fab fragment" means an N-terminal product resulting from the papain digestion of the antibody, and a fragment having a similar domain structure to such a fragment. The term "F(ab')₂ fragment" means a fragment obtained by reducing the disulfide bonds in the hinge region of the F(ab')₂ of the antibody, and a fragment having a similar domain structure to such a fragment. The term "F(ab)₂ fragment" means a dimer of two Fab fragment molecules which are bound to each other via disulfide bonds. The term "Fv fragment" means a minimum fragment which is a fragment of an antibody and is capable of binding to an antigen.

Examples of the antigen-binding region fragment of an antibody include, in more detail, antibodies to a protein which is specifically expressed in a particular cancer cell and Fab fragments or Fv fragments of such antibodies.

Examples of the antibody to a protein which is specifically expressed in a particular cancer cell include monoclonal antibodies, such as anti-CD25 antibodies and anti-CD20 antibodies.

Other embodiments of the protein or the peptide include cyclic pentapeptides which have affinity for integrins observed to be highly expressed in new blood vessels in cancer, such as cyclo-Arg-Gly-Asp-DPhe-Lys (SEQ ID NO: 1); F-Met-Leu-Phe (fMLP), a peptide having affinity for a receptor of a chemotactic factor present on the surface of macrophages;α-melanocyte stimulating hormone derivatives, which are peptides having affinity for a melanocortin receptor highly expressed in malignant melanoma; and somatostatin derivatives having affinity for a somatostatin receptor highly expressed in endocrine tumors.

Examples of the α-melanocyte stimulating hormone derivative include peptides having the sequence of Gly-Gly-Nle-cyclo (Asp-His-DPhe-Arg-Trp-Lys) (SEQ ID NO: 2). Examples of the somatostatin derivative include peptides having the sequence of DPhe-Cys-Tyr-DTrp-Lys-Thr-Cys-Thr (a disulfide bond is present between Cys2-Cys7) (SEQ ID NO: 3).

Note that F represents a formyl group, Nle represents norleucine, DPhe represents D-phenylalanine, and DTrp represents D-tryptophan.

The term "target molecule recognition element" refers to a monovalent group derived from a molecule capable of recognizing a target molecule in an organism, such as one which binds to (preferably, specifically binds to) the target molecule, an organic or inorganic monovalent group, and the like. However, the protein or the peptide described above are not included. The term includes, for example, a monovalent group derived from imidathiazole sulfonamide for carbonic anhydrase IX that is highly expressed in hypoxic regions of solid tumors, and a monovalent group derived from an asymmetric urea compound for a prostate-specific membrane antigen.

In the present specification, the term "target molecule" refers to a molecule that is a target of diagnosis using a radioactive drug and is present, preferably being specifically expressed, in a target site (for example, tissue or cell). The combination of the "target molecule" and the "target molecule recognition element" can be appropriately selected based on, for example, Binding DB (www.bindingdb.org/rwd/bind/index.jsp).

L¹ is a single bond or a linker group.

Examples of specific embodiments of the structure -NR²-L¹-Y in the above formula (1) include the following embodiments (w1) to (w6):
(w1) a structure -NR²-P that is a structure in which L¹ is a single bond and Y is a monovalent group P derived from a protein or a peptide;
(w2) a structure -NHR² that is a structure in which L¹ is a single bond and Y is a hydrogen atom;
(w3) a structure -NR²-(target molecule recognition element) that is a structure in which L¹ is a single bond and Y is a target molecule recognition element;
(w4) a structure -NR²-L¹-P that is a structure in which L¹ is a linker group and Y is a monovalent group P derived from a protein or a peptide;
(w5) a structure -NR²-L¹-H that is a structure in which L¹ is a linker group and Y is a hydrogen atom; and
(w6) a structure -NR²-L¹-(target molecule recognition element) that is a structure in which L¹ is a linker group and Y is a target molecule recognition element.

In the structure -NR²-P, the monovalent group P derived from a protein or a peptide is directly bound to the -NR² group, preferably via an amide bond.

In the structure -NR²-(target molecule recognition element), the target molecule recognition element is directly bound to the -NR² group, preferably via an amide bond.

The structure -NR^{Z}-L¹-H and the structure -NR²-L¹-P that are structures in which L¹ is a linker group are preferably formed by various kinds of protein crosslinking agents. In other words, the linker group of L¹ above is a linker group derived from the various kinds of protein crosslinking agents or a linker group having the various kinds of protein crosslinking agents as a part.

Specific examples of the crosslinking agent include protein crosslinking agents having a carboxyl-amine reactive group, such as a carbodiimide group; protein crosslinking agents having an amine reactive group, such as an N-hydroxysuccinimide (NHS) ester group or an imide group; and protein crosslinking agents having a carbonyl reactive group, such as a hydrazide group or an alkoxy amine group.

Further, the structure -NR²-L¹-P may be a structure containing a thioether group formed by a sulfhydryl reactive group, such as a maleimide group, and a sulfhydryl group derived from a sulfhydryl group introducing reagent, such as 2-iminothiolane.

The structure -NR²-L¹-(target molecule recognition element) that is a structure in which L¹ is a linker group is preferably formed by various kinds of crosslinking agents. Examples of such a structure include structures derived from crosslinking agents, including, for example, mercaptocarboxylic acids, such as 3-mercaptopropionic acid; and poly(ethylene glycol) 2-mercaptoethyl ether acetic acid, in addition to the protein crosslinking agents described above.

X¹ is a hydrogen atom or a pharmaceutically acceptable cation.

When X¹ is a pharmaceutically acceptable cation, the compound (1) forms a salt.

Examples of the pharmaceutically acceptable cation include inorganic cations, such as a sodium ion, a potassium ion, a calcium ion, a magnesium ion, and an ammonium ion; and organic cations, such as a triethylammonium ion, a triethanolammonium ion, a pyridinium ion, and a diisopropylammonium ion.

Examples of the pharmaceutically acceptable salt of the compound represented by the above formula (1) include acid addition salts and base addition salts.

The acid addition salt may be either an inorganic acid salt or an organic acid salt. Examples of the inorganic acid salt include, for example, hydrochlorides, hydrobromides, sulfates, hydroiodides, nitrates, and phosphates. Examples of the organic acid salt include, for example, citrates, oxalates, acetates, formates, propionates, benzoates, trifluoroacetates, maleates, tartrates, methanesulfonates, benzenesulfonates, and paratoluenesulfonates.

The base addition salt may be either an inorganic base salt or an organic base salt. Examples of the inorganic base salt include, for example, sodium salts, potassium salts, calcium salts, magnesium salts, and ammonium salts. Examples of the organic base salt include, for example, triethylammonium salts, triethanolammonium salts, pyridinium salts, and diisopropylammonium salts.

The compound of the present invention may be a solvate, such as a hydrate. The solvent is not particularly limited, as long as it is a pharmaceutically acceptable solvent.

The compound (1) or the like can be suitably used as a raw material for preparing a radiolabeled pharmaceutical containing the following compound (2) or the like as an active ingredient.

### <Compound (2) or the Like>

Hereinafter, a compound represented by the following formula (2) or a pharmaceutically acceptable salt thereof (hereinafter, also referred to simply as "compound (2) or the like") according to the present invention will be described in detail: 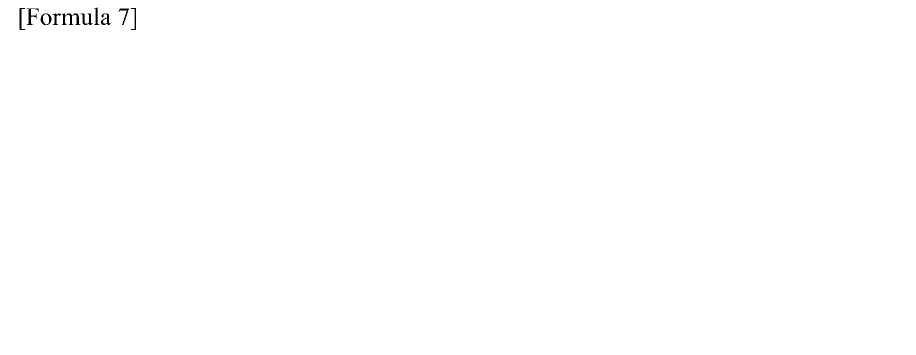 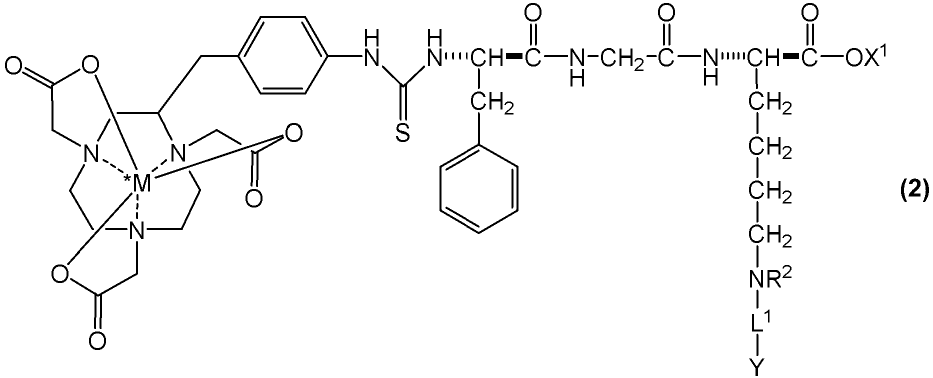 wherein
*M is ⁶⁷Ga, ⁶⁸Ga, ¹⁸F-Al, ⁶⁴Cu, or ⁶⁷Cu,
R² is a hydrogen atom or an organic group having 1 to 8 carbon atoms,
L¹ is a single bond or a linker group,
Y is a monovalent group P derived from a protein or a peptide, a hydrogen atom, or a target molecule recognition element,
X¹ is a hydrogen atom or a pharmaceutically acceptable cation, and
R² and L¹ are capable of binding to each other to form a nitrogen-containing heterocycle having 3 to 10 carbon atoms together with the adjacent nitrogen atom.

R², L¹, Y, and X¹ are the same as in the compound (1) or the like described above.

In the compound (2) or the like, a radioactive metal *M is coordinated to the 1,4,7-triazacyclononane-1,4,7-triacetic acid (hereinafter also referred to as "NOTA") part of the compound (1). In other words, the compound (2) or the like is a coordination compound (also referred to as a "complex") in which the radioactive metal *M is coordinated to the compound (1) as a ligand.

In the above formula (2), the dashed line represents a coordinate bond.

In the present specification, the radioactive metal *M includes not only metal elements which are radioactive isotopes, but also metal elements labeled with a radioactive compound, and is specifically ⁶⁷Ga, ⁶⁸Ga, ¹⁸F-Al, ⁶⁴Cu, or ⁶⁷Cu.

¹⁸F-Al is Al labeled with ¹⁸F, and can be introduced into the compound (2) or the like, for example, by the method described in [Bioconjugate Chem., 2014, 25, 2038-2045].

*M is preferably ⁶⁷Ga, ⁶⁸Ga, or ¹⁸F-Al, and more preferably ⁶⁷Ga or ⁶⁸Ga.

The compound (2) or the like can be suitably used as an active ingredient of a radiolabeled pharmaceutical.

### [Production Method]

### <Compound (1) or the Like >

The compound represented by the above formula (1) or a pharmaceutically acceptable salt thereof according to the present invention can be produced, for example, by one of the method (I) including the following steps (a1) to (a2) and the method (II) including the following steps (b1) to (b2).
{method (I)}
   (a1) a step of reacting an isothiocyanate group of a compound represented by the following formula (i) with an N-terminus of a phenylalanine residue of a compound represented by the following formula (ii);
   (a2) a step of reacting a linker group L^{1a} introduced into an amino group in a side chain of a lysine residue of the compound obtained in the step (a1), or the amino group, with a linker group L^{1b} or a molecular end of P of a compound represented by the following formula (iii), and
{method (II)}
   (b1) a step of reacting the linker group L^{1b} or the molecular end of P of the compound represented by the following formula (iii) with the linker group L^{1a} introduced into the amino group in the side chain of the lysine residue of the compound represented by the following formula (ii), or a step reacting the molecular end of P of the compound represented by the following formula (iii) with the amino group in the side chain of the lysine residue of the compound represented by the following formula (ii);
   (b2) a step of reacting the amino group of the phenylalanine residue at the N-terminus of the compound obtained in the step (b1) with the isothiocyanate group of the compound represented by the following formula (i): 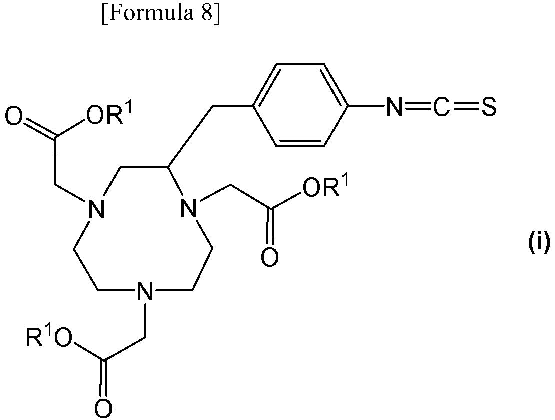
   wherein R¹s are each independently a hydrogen atom or a hydrocarbon group having 1 to 8 carbon atoms, 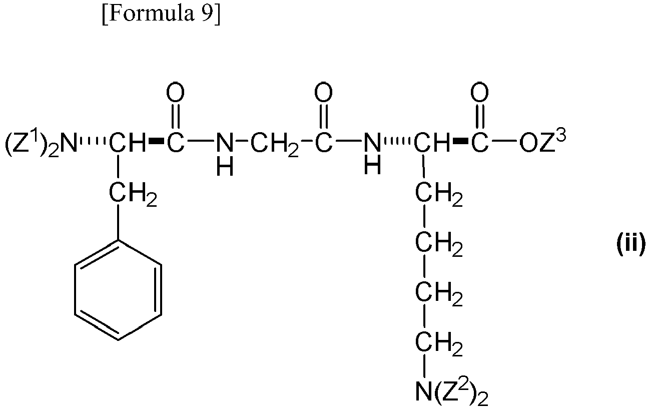
   wherein Z¹ and Z² are each independently a hydrogen atom or a protecting group of an amino group, and
   Z³ is a hydrogen atom, a protecting group of a carboxy group, or a bond with a solid phase,
      [Formula 10]

      (L^{1b})ₘ-P **(iii)**
   wherein m is 0 or 1,
   P is a monovalent group derived from a protein or a peptide,
   m is 0 or 1,
   when m is 0, P is a protein or a peptide, and
   when m is 1, L^{1b} is a linker group and P is a monovalent group derived from a protein or a peptide.

### {Method (1)}

### <Step (a1)>

In the step (a1), the isothiocyanate group of the compound represented by the above formula (i) is reacted with the N-terminus of the phenylalanine residue of the compound represented by the above formula (ii).

The compound represented by the above formula (i) can be produced by a known synthesis method. Further, commercially available products can also be used. Such commercially available products can be purchased from, for example, Macrocyclics (Texas, US).

The compound represented by the above formula (ii) can be produced by a known method of synthesizing a peptide.

Z¹ and Z² are each independently a hydrogen atom or a protecting group of an amino group. Examples of the protecting group of an amino group include a tert-butoxycarbonyl group (Boc group), a benzyloxycarbonyl group (Cbz group), and 9-fluorenylmethyloxycarbonyl (Fmoc group).

Z³ is a hydrogen atom, a protecting group of a carboxy group, or a bond with a solid phase. Examples of the protecting group of a carboxy group include a methyl group, an ethyl group, a benzyl group, and a tert-butyl group. The solid phase is preferably a solid phase used in a known method of synthesizing a peptide, including, for example, polymers, such as polystyrene, polyvinylidene fluoride, and nylon; glass surfaces; and cellulose derivatives, such as cellulose and nitrocellulose. The binding to the solid phase may be via a linker, if necessary.

In the reaction, for example, 0.1 mole to 10 moles, preferably 0.5 mole to 2 moles, and more preferably an approximately equimolar amount of the compound represented by the above formula (ii) is reacted with 1 mole of the compound represented by the above formula (i).

The reaction can be carried out in the presence of, for example, 0.1 mole to an excess molar amount, preferably 0.5 mole to 10 moles, of a base relative to 1 mole of the compound represented by the above formula (i), if necessary. Examples of the base include organic bases, such as pyridine, triethylamine, diisopropylethylamine (DIPEA), and 2,6-lutidine; and inorganic bases including alkali metal carbonates, such as sodium carbonate, alkali metal hydrogen carbonates, such as sodium bicarbonate, and alkali metal hydrides, such as sodium hydride. Among these, from the perspective of reactivity, organic bases are preferable. Further, from the perspective of capability of also acting as a solvent, organic bases which are liquid at room temperature are more preferable.

The reaction temperature and the reaction time can be appropriately set by those skilled in the art. The reaction temperature can be, for example, 0 to 40°C. The reaction time can be, for example, 30 minutes to ten days.

From the perspective of reaction progress, the reaction is preferably carried out in a solvent. Examples of the solvent include, but are not limited to, organic solvents, such as ethyl acetate, N,N-dimethylformamide (DMF), dimethyl sulfoxide (DMSO), 1,4-dioxane, tetrahydrofuran (THF), acetonitrile, and dichloromethane. The solvent may be either a single solvent or a mixed solvent containing two or more solvents.

### <Step (a2)>

In the step (a2), the linker group L^{1a} introduced into the amino group in the side chain of the lysine residue of the compound obtained in the step (a1), or the amino group, is reacted with the linker group L^{1b} or the molecular end of P of the compound represented by the above formula (iii).

The compound obtained by introducing the linker group L^{1a} into the amino group in the side chain of the lysine residue of the compound obtained in the step (a1) has a structure represented by the following formula (iv): 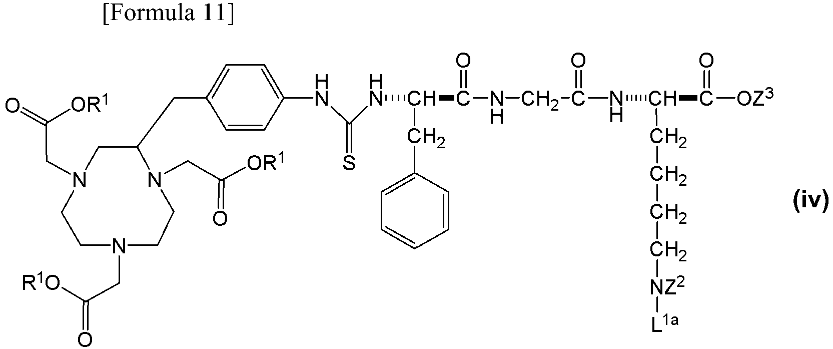 wherein R¹, Z², and Z³ are the same as those described above.

In the step (a2), when m=1, the linker group L^{1b} of the compound represented by the above formula (iii) reacts with the linker group L^{1a} introduced into the amino group in the side chain of the lysine residue of the compound obtained in the step (a1) or with the amino group. When m=0, the molecular end of P of the compound represented by the above formula (iii) reacts with the linker group L^{1a} introduced into the amino group in the side chain of the lysine residue of the compound obtained in the step (a1) or with the amino group.

The step (a2) can be carried out in accordance with a known method of crosslinking two proteins or peptides using a protein crosslinking agent. Examples of the protein crosslinking agent used include 2-iminothiolane.

When the amino group in the side chain of the lysine residue of the compound obtained in the step (a1) is reacted with the molecular end of P of the compound represented by the above formula (iii), the molecular end is preferably a carboxy group, and a peptide group is formed by the reaction.

When Z¹ and Z² are protecting groups of an amino group, the protecting groups can be deprotected at any stage by a usual method.

Examples of such a method include a method using, for example, an acid catalyst including an organic acid, such as trifluoroacetic acid and p-toluenesulfonic acid, and an inorganic acid, such as hydrochloric acid and sulfuric acid. Among them, the acid catalyst is preferably trifluoroacetic acid.

The reaction temperature and the reaction time can be appropriately set by those skilled in the art. The reaction temperature can be, for example, about 10 to 40°C. The reaction time can be, for example, about 30 minutes to 24 hours.

From the perspective of reaction progress, the reaction is preferably carried out in a solvent. Examples of the solvent include aqueous solvents, such as water.

### {Method (II)}

### <Step (b1)>

In the step (b1), the linker group L^{1b} or the molecular end of P of the compound represented by the above formula (iii) is reacted with the linker group L^{1a} introduced into the amino group in the side chain of the lysine residue of the compound represented by the above formula (ii), or the molecular end of P of the compound represented by the above formula (iii) is reacted with the amino group in the side chain of the lysine residue of the compound represented by the above formula (ii).

The compound obtained by introducing the linker group L^{1a} into the amino group in the side chain of the lysine residue of the compound represented by the above formula (ii) has a structure represented by the following formula (v): 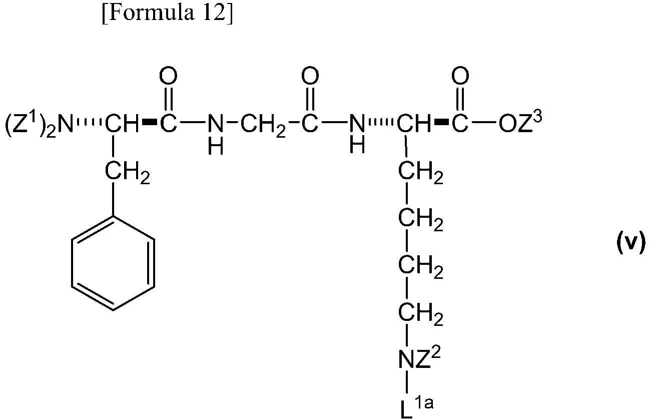 wherein Z¹, Z², and Z³ are the same as those described above.

The step (b1) can be carried out in the same manner as in the step (a2).

In the step (b1), when m=1, the linker group L^{1b} of the compound represented by the above formula (iii) reacts with the linker group L^{1a} introduced into the amino group in the side chain of the lysine residue of the compound represented by the above formula (ii) or with the amino group. When m=0, the molecular end of P of the compound represented by the above formula (iii) reacts with the linker group L^{1a} introduced into the amino group in the side chain of the lysine residue of the compound represented by the above formula (ii) or with the amino group.

### <Step (b2)>

In the step (b2), the amino group of the phenylalanine residue at the N-terminus of the compound obtained in the step (b1) is reacted with the isothiocyanate group of the compound represented by the above formula (i).

The step (b2) can be carried out in the same manner as in the step (a1).

Thus, the compound (1) or the like is produced by the method (I) or the method (II). The synthesis of the compound (1) or the like of the present invention can be confirmed by a known measure, such as ¹H-NMR measurement, ¹³C-NMR measurement, and mass spectrometry.

When Z³ is a bond with a solid phase, cleavage from the solid phase can be carried out at any stage by a known method.

### <Compound (2) or the Like>

The compound represented by the above formula (2) or a pharmaceutically acceptable salt thereof according to the present invention can be produced, for example, by a method including a step of reacting the compound represented by the above formula (1) or a pharmaceutically acceptable salt thereof with a salt of ⁶⁷Ga, ⁶⁸Ga, ¹⁸F-Al, ⁶⁴Cu, or ⁶⁷Cu.

Examples of the salt of ⁶⁷Ga, ⁶⁸Ga, ¹⁸F-Al, ⁶⁴Cu, or ⁶⁷Cu include halides, such as chlorides.

Examples of a specific method include simple operations using a conventionally known complex formation reaction.

### [Composition and the Like]

### <Composition for Preparing Radiolabeled Pharmaceutical>

The composition for preparing a radiolabeled pharmaceutical according to the present invention contains the above compound (1) as an essential ingredient.

The composition for preparing a radiolabeled pharmaceutical may contain, in addition to the above compound (1), a pH adjusting agent, such as an aqueous buffer solution, a stabilizing agent, such as ascorbic acid and p-aminobenzoic acid, and the like.

When Y is a hydrogen atom, the protein or the peptide described above can include the bifunctional protein crosslinking agents described above.

Further, the salt of ⁶⁷Ga, ⁶⁸Ga, ¹⁸F-Al, ⁶⁴Cu, or ⁶⁷Cu described above can be included.

The composition for preparing a radiolabeled pharmaceutical can be provided as a kit including the ingredients as separate packaging units.

The composition for preparing a radiolabeled pharmaceutical according to the present invention can be used in producing the compound (2) described above, and it is preferably used for preparing a radiolabeled pharmaceutical in the field of medical treatment, examination, and the like.

In relation to the embodiments described above, the present invention also encompasses the following embodiments:
- The compound (1) for preparing a radiolabeled pharmaceutical; and
- Use of the compound (1) for preparing a radiolabeled pharmaceutical.

### <Radiolabeled Pharmaceutical>

The radiolabeled pharmaceutical according to the present invention contains the above compound (2) as an essential ingredient.

The radiolabeled pharmaceutical can be prepared as a pharmaceutical composition containing one or two or more pharmaceutically acceptable carriers (carriers for pharmaceuticals), if necessary. Examples of the carriers for pharmaceuticals include, a pH adjusting agents, such as an aqueous buffer solution, an acid, and a base; a stabilizing agent, such as ascorbic acid and p-aminobenzoic acid; an excipient, such as D-mannitol; a tonicity agent; and a preservative. Further, compounds useful for improving radiochemical purity, such as citric acid, tartaric acid, malonic acid, sodium gluconate, and sodium glucoheptonate, may also be added. The radiolabeled pharmaceutical of the present invention can be provided in any of the forms of an aqueous solution, a frozen solution, and a lyophilized product.

The radiolabeled pharmaceutical of the present invention is used as, for example, a radiolabeled pharmaceutical for use in radiographic diagnostic imaging.

Examples of the radiographic diagnostic imaging include, for example, Single Photon Emission Computed Tomography (hereinafter, also referred to simply as "SPECT") and Positron Emission Tomography (hereinafter, also referred to simply as "PET").

The diagnosis is not particularly limited, and it is used in radiographic diagnostic imaging of various diseases, such as tumors, inflammation, infectious diseases, cardiovascular diseases, and brain/central system diseases, and various organs and tissues, and is preferably used in radiographic diagnostic imaging of tumors.

By selecting the protein or the peptide, the target molecule recognition element, and the like according to the characteristics of the target to be diagnosed, it is possible to diagnose or treat a wide variety of targets, and the radiolabeled pharmaceutical of the present invention can be widely used in the field of diagnosis.

Examples of a route of administration of the radiolabeled pharmaceutical of the present invention include, for example, parenteral administration, such as intravenous administration and intraarterial administration, and oral administration, and the intravenous administration is preferable.

The administration route is not limited to these routes, and any route can be used as long as the action of the radiolabeled pharmaceutical can be effectively exhibited after administration thereof.

The radioactivity intensity of the radiolabeled pharmaceutical of the present invention may be arbitrary, as long as the object can be achieved by administering the pharmaceutical and the clinical dose of the pharmaceutical is such that the radiation exposure of the subject is as low as possible.

The radioactivity intensity can be determined by referring to the radioactivity intensity used in a general diagnosing method or treating method using a radiolabeled pharmaceutical. Regarding the dosage, the amount of radioactivity and the dosage considered to allow the imaging are determined in view of various conditions, such as the age and the body weight of a patient, an appropriate radiographic imaging device, and the condition of the target disease.

For a human subject, the amount of radioactivity of various metals are as follows.

When ⁶⁷Ga is used, it is generally assumed to be used for SPECT, and the dosage of the diagnostic pharmaceutical is not particularly limited, and is, for example, 1.1 MBq/kg to 1.5 MBq/kg in terms of the amount of radioactivity of ⁶⁷Ga.

When ⁶⁸Ga is used, it is generally assumed to be used for PET, and the dosage of the diagnostic pharmaceutical is 1.5 MBq/kg to 3 MBq/kg in terms of the amount of radioactivity of ⁶⁸Ga.

When ¹⁸F-Al is used, it is generally assumed to be used for PET, and the dosage of the diagnostic pharmaceutical is 1.5 MBq/kg to 4 MBq/kg in terms of the amount of radioactivity of ¹⁸F.

When ⁶⁴Cu is used, it is generally assumed to be used for PET and radiation therapy, and the dosage of the diagnostic pharmaceutical is 1.5 MBq/kg to 4 MBq/kg in terms of the amount of radioactivity of ⁶⁴Cu, and 0.93 GBq/m² to 2.22 GBq/m² in terms of the amount of radioactivity of the radiation therapy.

When ⁶⁷Cu is used, it is generally assumed to be used for radiation therapy, and the dosage of the diagnostic pharmaceutical is 0.93 GBq/m² to 2.22 GBq/m² in terms of the amount of radioactivity of ⁶⁷Cu.

As described above, according to the present invention, a compound can be provided which achieves a radiolabeled pharmaceutical capable of reducing the accumulation in the kidney from the early stage after the administration and avoiding subsequent increase of the kidney accumulation. Thus, the radiolabeled pharmaceutical of the present invention can provide a clearer image and enables radiographic diagnostic imaging with higher accuracy than conventional ones. Further, since the accumulation in the kidney can be reduced, it is also possible to minimize a damage to other organs.

In relation to the embodiments described above, the present invention also encompasses the following embodiments:
- The compound (2) for preparing a radiolabeled pharmaceutical;
- Use of the compound (2) for preparing a radiolabeled pharmaceutical; and
- A method of diagnosing or treating a subject, including the steps of administering the compound (2) to a subject in need thereof, and measuring radiation in the subject based on the compound (1).

### Examples

Examples of the present invention described below are only for the purpose of illustration, and do not limit the technical scope of the present invention.

In the Examples and the Comparative Examples below, the following abbreviations for substituent groups, compounds, and organic solvents are used:
Fmoc: fluorenylmethoxycarbonyl group
Boc: tert-butoxycarbonyl group
Trt(2-Cl): 2-chlorotrityl group
p-SCN-Bn-NOTA: 2-S-(4-isothiocyanatobenzyl)-1,4,7-triazacyclononane-1,4,7-triacetic acid
Cl-Trt(2-Cl) Resin: 2-chlorotrityl chloride resin
Fmoc-Lys(Dde)-OH: N-α-(9-fluorenylmethoxycarbonyl)-N-ε-[1-(4,4-dimethyl-2,6-dioxocyclohexylidene)ethyl]-L-lysine
Fmoc-Lys-OH·HCl: N-α-(9-fluorenylmethoxycarbonyl)-L-lysine hydrochloride
TFA: trifluoroacetic acid
MeCN: acetonitrile
DIPEA: N,N-diisopropylethylamine
DMF: dimethylformamide
DIC: N,N'-diisopropylcarbodiimide
HOBt: 1-hydroxybenzotriazole
NMCM: N-methoxycarbonyl maleimide
EDTA: ethylenediaminetetraacetic acid
DPS: 2,2'-dipyridyl disulfide
EtOH: ethanol
FBS: fetal bovine serum
D-PBS: Dulbecco's phosphate-buffered saline
EGTA: glycol ether diamine tetraacetic acid
MGTA: DL-2-mercaptomethyl-3-guanidinoethylthiopropanoic acid.

### [Measuring Method and Experimental Animals]

In the Examples and the Comparative Examples below, the methods of measuring various physical properties or the like were as follows.

### <NMR (Nuclear Magnetic Resonance)>

The ¹H-NMR analysis was carried out using a JEOL ECS-400 spectrometer (JEOL Ltd.).

### <Thin Layer Chromatography (TLC)>

For analysis by thin layer chromatography (TLC), silica plates (TLC aluminum sheets Silica gel 60 RP-18 F₂₅₄s, MERCK, Tokyo, Japan) were used, and 10 cm of the plate was developed with a developing solvent, 0.1 M ammonium acetate aqueous solution: methanol (1:1), and cut into 5 mm pieces. The radioactivity of each fraction was measured using an Autowell gamma system (WIZARD3, PerkinElmer, USA).

### <Reverse-phase High-performance Liquid Chromatography (RP-HPLC) and Size Exclusion HPLC (SE-HPLC)>

Analysis by reverse-phase high-performance liquid chromatography (RP-HPLC) was carried out using an L-7405 (HITACHI Ltd., Tokyo, Japan) UV detector, an L-7100 (HITACHI Ltd.) liquid feeding pump, and a Cosmosil 5C₁₈-AR-300 column (4.6 mm x 150 mm, Nacalai Tesque Inc., Kyoto, Japan) as an analytical column. Fractionation by RP-HPLC was carried out using a Cadenza 5CD-C18 column (20 mm x 150 mm, Imtakt) connected to a guard column, a Cadenza 5CD-C18 guard column (10 mm x 8 mm, Imtakt).

The mobile phases used were 0.1% (v/v) TFA/H₂O (phase A) and 0.1% (v/v) TFA/MeCN (phase B). Elution was carried out at a flow rate of 5.0 mL/min. using a linear gradient method in which 90% (v/v) phase A: 10% (v/v) phase B was changed to 20% (v/v) phase A: 80% (v/v) phase B from 0 to 30 minutes, and then 20% (v/v) phase A: 80% (v/v) phase B was changed to 0% (v/v) phase A: 100% (v/v) phase B from 30 to 40 minutes.

Analysis by size exclusion HPLC (hereinafter, also referred to as "SE-HPLC") was carried out using a Cosmosil 5 Diol-300II (7.5 mm x 600 mm, Nacalai Tesque Inc., Kyoto, Japan) connected to a Cosmosil 5 Diol-300II guard column (7.5 mm x 50 mm, Nacalai Tesque Inc., Kyoto, Japan), and elution was carried out at a flow rate of 1.0 mL/min. using a 0.1 M phosphate buffer solution (pH 6.8) as the mobile phase.

### <Gamma Ray Detector>

For analysis of ⁶⁷Ga-labeled compounds, a gamma ray detector Gabi star (manufactured by Raytest) was connected online, or the eluate was fractionated at intervals of 0.5 minute using a fraction collector Frac-920 (manufactured by GE Healthcare Japan), and then radioactivity was measured using an Autowell Gamma System WIZARD3 (manufactured by Perkin Elmer).

### <Animal Experiments>

Animal experiments were carried out using male, six weeks old ddY SPF mice and male, eight to ten weeks old BALB/c-nu/nu mice (purchased from Japan SLC), as well as male Wistar rats. Further, the animal experiments were carried out with the approval of the Animal Ethics Committee of Chiba University.

### Synthesis Example 1: Synthesis of NOTA-Phe-Gly-Lys(Mal)

In accordance with the synthetic route below, the tripeptide sequence Phe-Gly-Lys synthesized by the Fmoc solid-phase synthesis method was maleoylated at its ε-amino group, and then reacted with p-SCN-NOTA under a basic condition to synthesize NOTA-Phe-Gly-Lys(Mal).

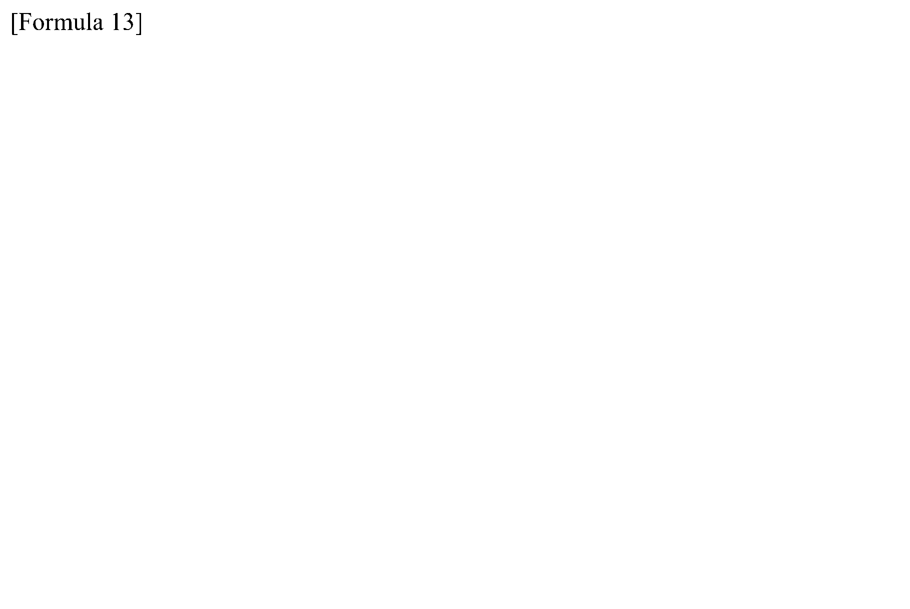

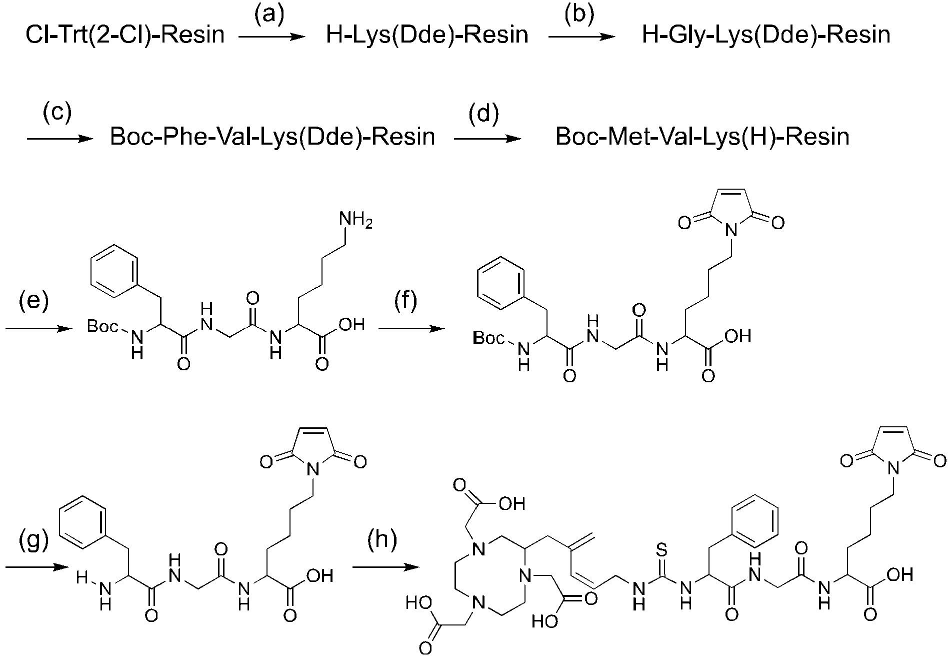

### (1-1) Synthesis of Boc-Phe-Gly-Lys-OH; Steps (a) to (e)

Cl-Trt(2-Cl)Resin (195 mg, 0.313 mmol), Fmoc-Lys(Dde)-OH (250 mg, 0.469 mmol), and DIPEA (327 µL, 1.876 mmol) were allowed to react in dichloromethane (2 mL) for 1.5 hours, and then methanol (1 mL) and DIPEA (327 µL, 1.876 mmol) were added to the mixture to quench the reaction. Subsequently, after washing with dichloromethane, 20% piperidine/DMF (3 mL) was added, and the solution was stirred at room temperature for 20 minutes to produce H-Lys(Dde)-Resin. A part of the resin was sampled to be subjected to a Kaiser test so that the deprotection of the Nα-Fmoc group was confirmed. The resin was washed with DMF and dichloromethane, dried, and then measured for the absorbance at A₃₀₁ of N-(9-fluorenylmethyl)piperidine formed in the piperidine treatment to determine the amount of Fmoc-Lys(Dde)-OH introduced into the resin (0.280 mmol, 89.5%). The H-Lys(Dde)-Resin (0.280 mmol) was stirred with 2.5 equivalents of Fmoc-Gly-OH (0.783 mmol), N,N'-diisopropylcarbodiimide (DIC, 0.783 mmol), and 1-hydroxybenzotriazole (HOBt, 0.783 mmol) in DMF (3 mL) at room temperature for two hours in accordance with the Fmoc solid-phase synthesis method. A part of the resin was sampled to be subjected to a Kaiser test so that the completion of the condensation reaction was confirmed, and then the resin was washed with DMF. Then, 20% piperidine/DMF (3 mL) was added thereto, and the mixture was stirred at room temperature for 20 minutes. The same procedure was carried out using Boc-Phe-OH (0.783 mmol) to produce Boc-Phe-Gly-Lys(Dde)-Resin, which was then stirred in 2% hydrazine/DMF (3 mL) at room temperature for one hour so that the Dde group was removed. The resin was washed with DMF and dichloromethane, and then stirred in a mixed liquid of acetic acid: 2,2,2-trifluoroethanol: dichloromethane (3:1:6, 2 mL) at room temperature for two hours. The resin was removed by filtration, and then the filtrate was distilled off under a reduced pressure. To the resulting residue, diethyl ether (3 mL) was added for crystallization. The crystals were collected by filtration, washed with diethyl ether, and then dried under a reduced pressure to obtain Boc-Phe-Gly-Lys-OH as white crystals.
ESI-MS m/z [M+H]⁺ 451, Found 451.

### (1-2) Synthesis of Boc-Phe-Gly-Lys(Mal)-OH; Step (f)

Boc-Phe-Gly-Lys-OH (65 mg, 0.14 mmol) was dissolved in saturated sodium bicarbonate aqueous solution (5 mL) under cooling with ice, and then N-(methoxycarbonyl)maleimide (NMCM) (33 mg, 0.21 mmol) was added to the solution. After stirring for 30 minutes under cooling with ice, the solution was stirred at room temperature for another 30 minutes. The solution was neutralized with 1 M sulfuric acid. The solution was extracted with chloroform (5 mL x 3), dried over sodium sulfate, and then the filtrate was distilled under a reduced pressure. The residue was purified by preparative TLC, so as to obtain Boc-Phe-Gly-Lys(Mal)-OH as white crystals.
ESI-MS m/z[M+H]⁺ 531, Found 531.

### (1-3) Synthesis of H-Phe-Gly-Lys(Mal)-OH; Step (g)

Boc-Phe-Gly-Lys(Mal)-OH (41 mg, 71.9 µmol) was dissolved in 3 mL of 4N HCl/ethyl acetate, and the solution was stirred at room temperature for one hour. The solvent was distilled off under a reduced pressure, and the residue was subjected to azeotropy with hexane, so as to obtain a hydrochloride of Phe-Gly-Lys(Mal)-OH as white crystals. ESI-MS m/z [M+H]⁺ 431, Found 431.

### (1-4) Synthesis of NOTA-Phe-Gly-Lys(Mal)-OH (NOTA-FGK-Mal); Step (h)

The hydrochloride of Phe-Gly-Lys(Mal)-OH (9.6 mg, 13 µmol) was dissolved in dry DMF (200 µL), and triethylamine (12 µL) was added to the solution. p-SCN-NOTA (9.7 mg, 21 µmol) was added to the solution, and the mixture was stirred at room temperature for two hours, then diluted ten times with Milli-Q water and purified by preparative RP-HPLC, so as to obtain NOTA-Phe-Gly-Lys(Mal)-OH as white crystals.
ESI-MS m/z [M+H]⁺ 881, Found 881.

### Synthesis Example 2: Synthesis of NOTA-Phe-Gly-Lys-Fab

### (2-1) Production of Anti-Fab Fragment

IgG (9 mg) was dissolved in 1.5 mL of a 20 mM phosphate buffer solution (pH 7.0) containing 10 mM Na₂EDTA and 20 mM cysteine, and 500 µL of an immobilized papain 50% slurry (manufactured by Thermo Scientific, Inc.) was added, and the mixture was incubated at 37°C for 42 hours. After the completion of the reaction, 2 mL of a 10 mM tris hydrochloride buffer solution (pH 7.5) was added thereto, the mixture was filtered with a filter of 0.45 µm, and the filtrate was recovered. The filtrate was replaced with a 20 mM phosphate buffer solution (pH 7.0) by using a 10 kDa ultrafiltration membrane, and concentrated to 1 mL. Subsequently, the concentrated filtrate was purified with the Protein A column, so as to obtain Fab. The production of the resulting Fab was confirmed by SE-HPLC eluting with a 0.1 M phosphate buffer solution (pH 6.8) as an eluting solvent at a flow rate of 1.0 mL/min., and the concentration of the Fab was calculated by measuring A₂₈₀.

### (2-2) Production of IT-Fab

A Fab solution (200 µL, 2.0 mg/mL) was prepared by using a sufficiently deaerated 0.16 M borate buffer solution (pH 8.0) containing 2 mM EDTA. To the solution was added 2-iminothiolane (2-IT) (7.5 µL, 2.0 mg/mL) dissolved in the same buffer solution, and the mixture was allowed to react at 37°C for 30 minutes. After the reaction, the excess 2-IT in the reaction solution was removed by the spin column method using Sephadex G-50 Fine equilibrated with a sufficiently deaerated 0.1 M phosphate buffer solution (pH 6.0) containing 2 mM EDTA. The number of thiol groups introduced into one molecule of Fab was measured with 2,2'-dipyridyl disulfide (DPS).

### (2-3) Production of NOTA-Phe-Gly-Lys-Fab

NOTA-Phe-Gly-Lys(Mal) (1.25 µL, 50 mg/mL) dissolved in DMF was added to the Fab solution (1.6 mg/mL, 95 µL) thiolated with 2-IT, and the solution was allowed to react at 37°C for one hour. After the reaction, the excess NOTA-Phe-Gly-Lys(Mal) in the reaction solution was removed by the spin column method using Sephadex G-50 Fine equilibrated with a sufficiently deaerated 0.1 M phosphate buffer solution (pH 6.0) containing 2 mM EDTA. Subsequently, an iodoacetamide solution (10 mg/mL) was prepared using 0.1 M phosphate buffer solution (pH 6.0) containing 2 mM EDTA, and 12.5 µL of the solution was added the reaction solution, which was then allowed to react at 37°C for one hour, so that unreacted thiol groups were alkylated. Subsequently, the product was purified by the spin column method using Sephadex G-50 Fine equilibrated with a 0.25 M acetate buffer solution (pH 5.5). The number of chelates introduced into one molecule of Fab was calculated by determining the amount of thiol groups using DPS before and after the reaction.

### Synthesis Example 3: Synthesis of ⁶⁷Ga-labeled NOTA-Phe-Gly-Lys-Fab

⁶⁷GaCl₃ (5 µL, manufactured by PDRadiopharma Inc.) was mixed with a 0.25 M acetate buffer solution (pH 5.5, 5 µL), and the mixture was allowed to stand at room temperature for five minutes. After mixing an NOTA-Phe-Gly-Lys-Fab solution (10 µL) therewith, the mixture was incubated at 37°C for one hour. After adding a 20 mM EDTA solution (20 µL) to the mixture, the product was purified by the spin column method using Sephadex G-50 Fine equilibrated with D-PBS (-), so as to produce ⁶⁷Ga-NOTA-Phe-Gly-Lys-Fab. The radiochemical yield of 95% or more was confirmed by TLC and SE-HPLC.

### Comparative Synthesis Example C1: Synthesis of NOTA-Met-Val-Lys(Mal)

NOTA-Met-Val-Lys(Mal) was synthesized in accordance with the description of PTL 1.

### Comparative Synthesis Example C2: Synthesis of ⁶⁷Ga-labeled NOTA-Met-Val-Lys-Fab

NOTA-Met-Val-Lys-Fab was produced in the same manner as in the Synthesis Example 2 except that NOTA-Met-Val-Lys(Mal) was used instead of the NOTA-Phe-Gly-Lys(Mal). Subsequently, ⁶⁷Ga-labeled NOTA-Met-Val-Lys-Fab was synthesized in the same manner as in the Synthesis Example 3 except that NOTA-Met-Val-Lys-Fab was used instead of the NOTA-Phe-Gly-Lys-Fab.

### Synthesis Example 4: Synthesis of NOTA-Phe-Gly-Lys(Bzo)

### (4-1) Synthesis of NOTA-Phe and NOTA-Phe-Gly

p-SCN-NOTA (5 mg, 0.011 mmol) was dissolved in a 0.1 M borate buffer solution (pH 9.0) so that the final concentration was from 10 to 100 mg/mL. The solution was adjusted to have a pH of 8 to 9 with a 0.1 N sodium hydroxide aqueous solution, and then Phe or Phe-Gly (0.011 mmol) was added thereto. The solution was allowed to react at room temperature for two hours. The product was purified by preparative RP-HPLC.
ESI-MS m/z NOTA-Phe: [M+H]⁺ 616, Found 616, NOTA-Phe-Gly: [M+H]⁺ 673, Found 673.

### (4-2) Synthesis of N-(Benzoyloxy)succinimide

Benzoic acid (1.0 g, 8.20 mmol) and N-hydroxysuccinimide (1.04 g, 9.02 mmol) were dissolved in 30 mL of dry DMF, and dicyclohexylcarbodiimide (1.86 g, 9.02 mmol) dissolved in 10 mL of dry DMF was added dropwise to the solution in an argon atmosphere at 0°C. The mixture was returned to room temperature, and stirred for 12 hours. The precipitate was then removed by filtration, and the solvent was distilled off under a reduced pressure, so as to obtain 2.06 g of a crude product of N-(Benzoyloxy)succinimide as a white solid matter. The compound contained a small amount of urea, but was used directly for the subsequent reaction.
¹H-NMR (CDCl₃): δ 2.91 (4H, t, CH₂), 8.00-8.24 (5H, m, Ar-H); ESI-MS m/z [M+H]⁺ 220, Found 220.

### (4-3) Synthesis of Fmoc-Lys(Bzo)-OH

N-(Benzoyloxy)succinimide (27 mg, 0.25 mmol) was dissolved in dichloromethane (1 mL), and added dropwise to Fmoc-Lys-OH·HCl (50 mg, 0.25 mmol) and sodium bicarbonate (20 mg, 0.50 mmol) dissolved in Milli-Q water (1 mL), and then the mixture was vigorously stirred at room temperature. After stirring for 24 hours, the mixture was made acidic with 1 N hydrochloric acid, and then extracted with dichloromethane (5 mL x 3), and the organic layer was dried over anhydrous magnesium sulfate. After distilling off the solvent under a reduced pressure, the residue was purified by open column chromatography with hexane: ethyl acetate: acetic acid (1: 2: 0.1) as an eluting solvent, so as to obtain 57 mg of an Fmoc-Lys(Bzo)-OH (0.12 mmol, 49%).
¹H-NMR (CDCl₃): δ 1.48-1.94 (6H, m, CH₂), 3.46 (2H, t, CH₂), 3.73-4.40 (4H, m, CH, CH₂), 7.23-8.06 (13H, m, Ar-H); ESI-MS m/z [M+H]⁺ 473, Found 473.14.

### (4-4) Synthesis of H-Phe-Gly-Lys(Bzo)

Cl-Trt(2-Cl)Resin (32 mg, 0.052 mmol), Fmoc-Lys(Bzo)-OH (37 mg, 0.078 mmol), and DIPEA (36 µL, 0.209 mmol) were allowed to react in dichloromethane (0.5 mL) for 1.5 hours, and then methanol (0.2 mL) and DIPEA (36 µL, 0.209 mmol) were added to the mixture to quench the reaction. After washing the resin with DMF and then with dichloromethane, 20% piperidine/DMF (3 mL) was added, and the solution was stirred at room temperature for 20 minutes to produce H-Lys(Bzo)-Resin. A part of the resin was sampled to be subjected to a Kaiser test so that the deprotection of the Nα-Fmoc group was confirmed. The resin was washed with DMF and dichloromethane, dried, and then measured for the absorbance at A₃₀₁ of N-(9-fluorenylmethyl)piperidine formed in the piperidine treatment to determine the amount of Fmoc-Lys(Bzo)-OH introduced into the resin (0.040 mmol, 76%). The H-Lys(Bzo)-Resin (0.026 mmol) was stirred with 2.5 equivalents of Fmoc-Gly-OH (0.065 mmol), N,N'-diisopropylcarbodiimide (DIC, 10 µL, 0.065 mmol), and 1-hydroxybenzotriazole (HOBt, 0.065 mmol) in DMF (3 mL) at room temperature for two hours in accordance with the Fmoc solid-phase synthesis method. A part of the resin was sampled to be subjected to a Kaiser test so that the completion of the condensation reaction was confirmed, and then the resin was washed with DMF. Then, 20% piperidine/DMF (3 mL) was added thereto, and the mixture was stirred at room temperature for 20 minutes. The same procedure was carried out using Fmoc-Phe-OH (0.065 mmol) to produce H-Phe-Gly-Lys(Bzo)-Resin, which was then stirred in a mixed liquid of acetic acid: 2,2,2-trifluoroethanol: dichloromethane (3:1:6, 2 mL) at room temperature for two hours. The resin was removed by filtration, and then the filtrate was distilled off under a reduced pressure. To the resulting residue, diethyl ether (3 mL) was added for crystallization. The crystals were collected by filtration, washed with diethyl ether, and then dried under a reduced pressure to obtain an acetate of H-Phe-Gly-Lys(Bzo) (9.7 mg, 69.0%) as white crystals. ESI-MS m/z [M+H]⁺ 455, Found 455.

### (4-5) Synthesis of NOTA-Phe-Gly-Lys(Bzo)

p-SCN-NOTA (5 mg, 11 µmol) was dissolved in a 0.1 M borate buffer solution (pH 9.0) so that the final concentration was from 10 to 100 mg/mL. The solution was adjusted to have a pH of 8 to 9 with a 0.1 N sodium hydroxide aqueous solution, and then added with H-Phe-Gly-Lys(Bzo) (5.0 mg, 11 µmol) was added thereto. The solution was allowed to react at room temperature for two hours. The product was purified by preparative RP-HPLC, so as to obtain 7.7 mg (77%) of NOTA-Phe-Gly-Lys(Bzo) as a white solid matter.
ESI-MS m/z [M+H]⁺ 905, Found 905.

### Synthesis Example 5: Synthesis of ⁶⁷Ga-labeled NOTA-Phe-Gly-Lys(Bzo)

⁶⁷GaCl₃ (1.0 µL) was mixed with a 0.1 M ammonium acetate buffer solution (pH 5.5, 10 µL), and the mixture was allowed to stand at room temperature for five minutes. After mixing an NOTA-Phe-Gly-Lys(Bzo) solution (2.0 x 10⁻⁴M, 10 µL), the mixture was allowed to react at 37°C for one hour, so as to synthesize ⁶⁷Ga-NOTA-Phe-Gly-Lys(Bzo). The production thereof was confirmed based on the fact that the retention time of the compound by RP-HPLC was identical to that of the non-radioactive Ga complex.

For the non-radioactive Ga complex, GaCl₃ (1.1 mg, 6.5 µmol) was dissolved in 0.25 M sodium acetate buffer solution (pH 5.5, 50 µL), and NOTA-Phe or NOTA-Phe-Gly (3 µmol) was added thereto. After reaction at room temperature for one hour, the product was purified by analytical RP-HPLC, so as to obtain non-radioactive Ga-NOTA-Phe and Ga-NOTA-Phe-Gly as white solid matters.
ESI-MS m/z Ga-NOTA-Phe: [M+H]⁺ 683, Found 683, Ga-NOTA-Phe-Gly: [M+H]⁺ 739, Found 739, Ga-NOTA-Phe-Gly-Lys-Bzo: [M+H]⁺ 971, Found 971.

### Comparative Synthesis Example C3: Synthesis of NOTA-Met-Val-Lys(Bzo)

NOTA-Met-Val-Lys(Bzo) was synthesized in accordance with the description of PTL 1.

### Comparative Synthesis Example C4: Synthesis of ⁶⁷Ga-labeled NOTA-Met-Val-Lys(Bzo)

⁶⁷Ga-labeled NOTA-Met-Val-Lys(Bzo) was synthesized in accordance with the description of PTL 1.

### Measurement Example 1: Test by Incubation with BBMVs (Kidney Brush Border Membrane Vesicles)

### (Preparation of BBMVs (Kidney Brush Border Membrane Vesicles))

Kidney brush border membrane vesicles (BBMVs) were prepared from the kidney of a male Wistar rat (200 to 250 g) in accordance with the method of Hori et al. [Biochemical Pharmacology 45: 1763-1768, 1993]. All the operations were carried out on ice. From four to five times the mass of 300 mM mannitol and a 12 mM tris hydrochloride buffer solution (pH 7.1) containing 5 mM EGTA were added to the cortex, and the mixture was homogenized with a Polytron Homogenizer PT-3100 (manufactured by Kinematica GmgH) for two minutes, and diluted with the same buffer solution, so as to obtain a 10% homogenate. Subsequently, the homogenate was diluted with distilled water two times, and an MgCl₂ aqueous solution adjusted to 1.0 M was added thereto so that the final concentration was 10 mM, followed by allowing to stand for 15 minutes. Subsequently, the homogenate was subjected to centrifugation at 1,900 g, and the supernatant was subjected to further centrifugation at 24,000 g for 30 minutes. The precipitate was resuspended in 150 mM mannitol and 6 mM tris hydrochloride buffer solution (pH 7.1) containing 2.5 mM EGTA in an amount corresponding to 20 times the mass of the cortex, and homogenized with a Teflon (TM) homogenizer (1,000 rpm, 10 strokes). Subsequently, a 1.0 M MgCl₂ aqueous solution was added to the suspension so that the final concentration was 10 mM, and the suspension was allowed to stand for 15 minutes. The homogenate was then subjected to centrifugation at 1,900 g, and the supernatant was subjected to further centrifugation at 24,000 g for 30 minutes. The resulting precipitate was suspended in a 0.1 M phosphate buffer solution (pH 7.0) in an amount corresponding to ten times the mass of the cortex, and homogenized again with the Teflon (TM) homogenizer (1,000 rpm, 10 strokes). Subsequently, the homogenate was subjected to centrifugation at 24,000 g for 30 minutes, so as to obtain BBMVs as a precipitate. Subsequently, the precipitate of BBMVs was resuspended in a 0.1 M phosphate buffer solution (pH 7.0) and passed through a needle of 0.4 mm x 19 mm for ten times, so that the sizes of the vesicles were constant. For the incubation test, the BBMVs were used after diluting to a protein concentration of 10 mg/mL. The BBMVs thus prepared were evaluated for the incorporation of the lysosome enzyme by measuring the activity of β-galactosidase as a lysosome marker enzyme using p-nitrophenyl-β-D-galacto-pyranoside [Plant Physiology 55: 94-98, 1975]. Further, the activities of γ -glutamyl transferase and aminopeptidase were measured by using L-γ-glutamyl-p-nitroanilide and L-leucine-p-nitroanilide in accordance with the methods of Glossmann et al. [FEBS Letters 19: 340-344, 1972], Kramers et al. [European Journal of Biochemistry 99: 345-351, 1979].

### (Incubation Test)

The incubation test of BBMVs and a ⁶⁷Ga-labeled low-molecular model substrate was carried out in the following manner. The BBMVs (10 µL) prepared to have a protein concentration of 10 mg/mL was pre-incubated at 37°C for 10 minutes. Subsequently, excess ligands were removed by reverse-phase HPLC, and a ⁶⁷Ga-labeled NOTA-Phe-Gly-Lys(Bzo) or ⁶⁷Ga-labeled NOTA-Met-Val-Lys(Bzo) solution (10 µL) dissolved in PBS was added to the BBMVs solution, which was incubated at 37°C for two hours. Ethanol was added to the BBMVs solution so that the ethanol concentration was 60%, and the mixture was subjected to centrifugation at 5,000 rpm for ten minutes. After the supernatant was recovered, 60% ethanol was added to the precipitate and centrifugation was carried out again in the same manner. Subsequently, the supernatant was recovered. The supernatant obtained was analyzed by HPLC at a flow rate of 1 mL/min. using an Imtakt Unison US-C18 150 mm x 4.6 mm column. As mobile phases, 0.1% TFA/Milli-Q water was used for the phase A, and 0.1% TFA/MeCN was used for the phase B. The analysis was carried out using a linear gradient method in which 100% phase A: 0% phase B was changed to 55% phase A: 45% phase B from 0 to 30 minutes.

Fig. 1 illustrates results of experiments in which ⁶⁷Ga-labeled NOTA-Met-Val-Lys(Bzo) (in Fig. 1, "MVK") or ⁶⁷Ga-labeled NOTA-Phe-Gly-Lys(Bzo) (in Fig. 1, "FGK") was incubated with the BBMVs. The numerical values in Fig. 1 are percentages (%) of the radioactivity of a metabolite produced with respect to the radioactivity in the incubated BBMVs as 100%.

A peak of ⁶⁷Ga-labeled-NOTA-Phe-Gly was observed, not a peak of ⁶⁷Ga-labeled-NOTA-Phe. The amount of ⁶⁷Ga-NOTA-Phe-Gly released was lower than the amount of ⁶⁷Ga-labeled-NOTA-Met released from ⁶⁷Ga-labeled NOTA-Met-Val-Lys(Bzo).

### Measurement Example 2: Measurement of Internal Kinetics in Mice

The ⁶⁷Ga-labeled NOTA-Phe-Gly-Lys-Fab prepared by the method described above was diluted with D-PBS (-). A ⁶⁷Ga-labeled Fab solution (0.3 µCi/100 µL/mouse) adjusted to have a Fab concentration of 5 µg/100 µL was administered to the mice via the tail vein. Five to three mice from each group were sacrificed ten minutes, one hour, three hours, and six hours after administration, and blood and organs of interest (liver, kidney, spleen, stomach, and intestine) were collected, their masses were measured, and the radioactivity was then measured using an Autowell Gamma System. Feces and urine were also collected up to six hours after administration, and the radioactivity in the feces and urine was measured using the Autowell Gamma System.

As a control, the internal kinetics of ⁶⁷Ga-labeled NOTA-Met-Val-Lys-Fab in mice was measured in the same manner.

The results are shown in Table 1.

**[Table 1]**

| | Elapsed time after administration | | | |
|---|---|---|---|---|
| | 10 minutes | 1 hour | 3 hours | 6 hours |
| | [⁶⁷Ga]-NOTA-FGK-Fab | | | |
| Blood | 23.05 ±2.34 | 9.93 ±1.31 | 3.85 ±0.44 | 2.15 ±0.16 |
| Liver | 3.68 ±0.43 | 1.92 ±0.08 | 1.16 ±0.13 | 0.86 ±0.19 |
| Kidney | 16.0 ±2.39 | 10.0 ±0.84 | 7.07 ±2.82 | 4.01 ±0.39 |
| Spleen | 3.04 ±0.58 | 1.52 ±0.30 | 0.76 ±0.11 | 0.44 ±0.07 |
| Stomach* | 0.46 ±0.03 | 0.77 ±0.30 | 0.48 ±0.06 | 0.34 ±0.10 |
| lntestine* | 2.56 ±0.30 | 5.50 ±0.72 | 7.63 ±1.31 | 8.90 ±2.32 |

| | [⁶⁷Ga]-NOTA-MVK-Fab | | | |
|---|---|---|---|---|
| Blood | 23.4 ±3.74 | 10.8 ±1.58 | 5.38 ±0.59 | 3.10 ±0.35 |
| Liver | 4.50 ±0.84 | 2.74 ±0.55 | 1.81 ±0.23 | 1.38 ±0.26 |
| Kidney | 16.0 ±2.83 | 15.7 ±2.72 | 8.82 ±1.21 | 6.02 ±0.87 |
| Spleen | 3.37 ±0.46 | 1.82 ±0.26 | 1.22 ±0.24 | 0.74 ±0.17 |
| Stomach* | 0.44 ±0.08 | 0.64 ±0.08 | 0.55 ±0.07 | 0.44 ±0.14 |
| Intestine* | 2.27 ±0.65 | 4.13 ±0.57 | 5.56 ±0.63 | 7.64 ±2.57 |

| | | | | |
|---|---|---|---|---|
| * Expressed as %ID | | | | |

The numerical values in the table indicate changes in internal radioactivity distribution over time. The units of the numerical values are the radioactivity accumulation rate (%) [%ID/g] relative to the injected dose per g of organ or tissue as 100%, for organs or tissues other than the stomach and the intestine, and the radioactivity accumulation rate (%) [%ID] relative to the injected dose per organ or tissue as 100%, for the stomach and the intestine.

A significant difference in the kidney radioactivity was observed between ⁶⁷Ga-labeled NOTA-Phe-Gly-Lys-Fab and ⁶⁷Ga-labeled NOTA-Met-Val-Lys-Fab. For ⁶⁷Ga-labeled NOTA-FGK-Fab, the radioactivity from the kidney decreased over time after the administration, and was significantly lower than that of ⁶⁷Ga-labeled NOTA-MVK-Fab at one hour after the administration.

On the other hand, the blood clearance exhibited similar results.

### Measurement Example 3: Measurement of Internal Kinetics in Mice

### (Cell Culture)

Brain tumor cells U87MG were cultured under the following conditions: a 150 mm Cell Culture Dish-Treated (manufactured by True Line) was used as the culture vessel; a DMEM medium (manufactured by Fujifilm Wako Pure Chemical Corporation) supplemented with 10% (v/v) FBS (manufactured by Nippon Bio-Supp. Center) and 1% (v/v) penicillin/streptomycin (5000 units-5000 µg/mL, manufactured by Invitrogen, Life Technologies Japan Ltd.) was used as the medium; and the cells were incubated at 37°C and under 5% (v/v) CO₂ and saturated water vapor pressure.

### (Production of Tumor Model Mice)

BALB/c-nu/nu mice (male, eight to ten weeks old) were subcutaneously transplanted with 3 x 10⁶ U87MG cells in the left thigh. After four to five weeks, tumor-bearing mice with the tumor size increased to 0.4 g to 1.0 g were obtained as tumor model mice.

### (Measurement of Internal Kinetics)

The ⁶⁷Ga-labeled NOTA-Phe-Gly-Lys-Fab and ⁶⁷Ga-labeled NOTA-Met-Val-Lys-Fab prepared by the method described above were diluted with phosphate buffered saline (1mM, pH 7.4). The respective radioactive metal-labeled Fab solutions (0.3 µCi/100 µL/mouse) adjusted to have a Fab concentration of 5 µg/100 µL was administered to the tumor model mice above via the tail vein. Five mice from each group were sacrificed one hour and three hours after administration, and blood and organs of interest were collected, their masses were measured, and the radioactivity was then measured using an Autowell Gamma System.

The results are shown in Table 2.

**[Table 2]**

| | Elapsed time after administration | | | |
|---|---|---|---|---|
| | 1 hour | 3 hours | 1 hour | 3 hours |
| | [⁶⁷Ga]-NOTA-FGK-Fab | | [⁶⁷Ga]-NOTA-MVK-Fab | |
| Blood | 13.61 ±0.68 | 5.64 ±0.89 | 17.48 ±0.83 | 8.10 ±0.98 |
| Liver | 7.05 ±0.44 | 3.72 ±0.52 | 8.86 ±0.55 | 5.64 ±0.52 |
| Kidney | 16.78 ±1.55 | 10.55 ±4.24 | 32.21 ±2.43 | 17.82 ±1.69 |
| Spleen | 2.26 ±0.29 | 1.56 ±0.83 | 3.22 ±0.32 | 1.54 ±0.17 |
| Stomach* | 1.35 ±0.61 | 0.40 ±0.13 | 0.71 ±0.18 | 0.59 ±0.06 |
| Intestine* | 9.63 ±0.97 | 19.32 ±1.78 | 9.63 ±0.87 | 14.92 ±1.94 |
| Tumor | 1.88 ±0.38 | 2.34 ±0.95 | 2.17 ±0.32 | 2.69 ±0.42 |

| | | | | |
|---|---|---|---|---|
| * Expressed as %ID | | | | |

The numerical values in the table indicate changes in internal radioactivity distribution over time. The units of the numerical values are the radioactivity accumulation rate (%) [%ID/g] relative to the injected dose per g of organ or tissue as 100%, for organs or tissues other than the stomach and the intestine, and the radioactivity accumulation rate (%) [%ID] relative to the injected dose per organ or tissue as 100%, for the stomach and the intestine.

A significant difference in the kidney radioactivity was observed between ⁶⁷Ga-labeled NOTA-Phe-Gly-Lys-Fab and ⁶⁷Ga-labeled NOTA-Met-Val-Lys-Fab. For ⁶⁷Ga-labeled NOTA-FGK-Fab, the radioactivity from the kidney decreased over time after the administration, and was significantly lower than that of ⁶⁷Ga-labeled NOTA-MVK-Fab at one hour and three hours after the administration.

On the other hand, the accumulation in the tumor exhibited similar results.

Although the reason why the effects of the present invention are achieved is not necessarily clear, it is considered that, for ⁶⁷Ga-labeled NOTA-Phe-Gly-Lys-Fab, ⁶⁷Ga-labeled NOTA-Phe-Gly is rapidly produced due to the enzymatic action on the kidney rush border membrane, and for ⁶⁷Ga-labeled NOTA-Phe-Gly, it has high urinary excretion.

## Claims

1. A compound represented by the following formula (1) or a pharmaceutically acceptable salt thereof: 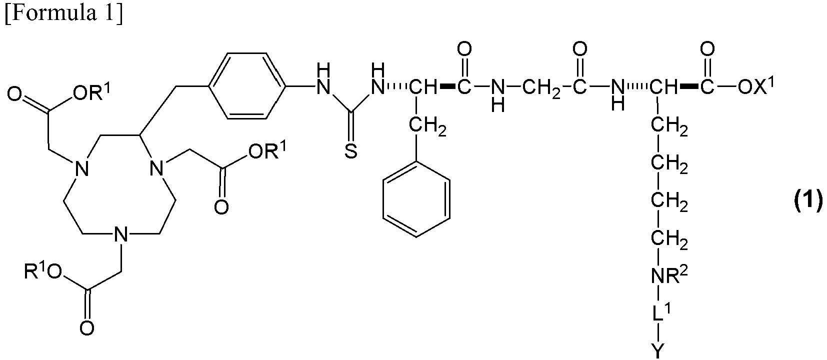 wherein
R¹s are each independently a hydrogen atom or a hydrocarbon group having 1 to 8 carbon atoms,
R² is a hydrogen atom or an organic group having 1 to 8 carbon atoms,
L¹ is a single bond or a linker group,
Y is a monovalent group P derived from a protein or a peptide, a hydrogen atom, or a target molecule recognition element,
X¹ is a hydrogen atom or a pharmaceutically acceptable cation, and
R² and L¹ are capable of binding to each other to form a nitrogen-containing heterocycle having 3 to 10 carbon atoms together with the adjacent nitrogen atom.

2. The compound or a pharmaceutically acceptable salt thereof according to claim 1, wherein Y is a monovalent group P derived from a protein or a peptide.

3. A composition for preparing a radiolabeled pharmaceutical, comprising the compound or a pharmaceutically acceptable salt thereof according to claim 1 or 2.

4. A compound represented by the following formula (2) or a pharmaceutically acceptable salt thereof: 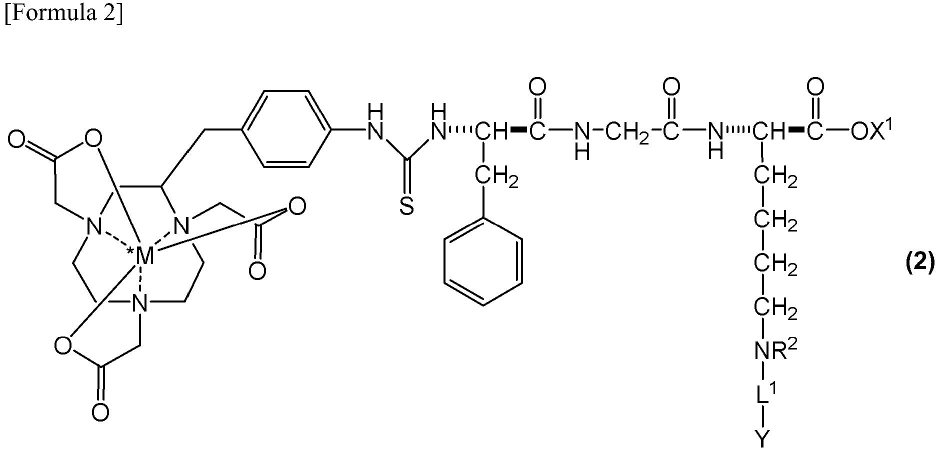 wherein
*M is ⁶⁷Ga, ⁶⁸Ga, ¹⁸F-Al, ⁶⁴Cu, or ⁶⁷Cu,
R² is a hydrogen atom or an organic group having 1 to 8 carbon atoms,
L¹ is a single bond or a linker group,
Y is a monovalent group P derived from a protein or a peptide, a hydrogen atom, or a target molecule recognition element,
X¹ is a hydrogen atom or a pharmaceutically acceptable cation, and
R² and L¹ are capable of binding to each other to form a nitrogen-containing heterocycle having 3 to 10 carbon atoms together with the adjacent nitrogen atom.

5. The compound or a pharmaceutically acceptable salt thereof according to claim 4, wherein Y is a monovalent group P derived from a protein or a peptide.

6. A radiolabeled pharmaceutical composition comprising the compound or a pharmaceutically acceptable salt thereof according to claim 4 or 5.

7. A method of producing the compound or a pharmaceutically acceptable salt thereof according to claim 1 or 2, the method being one of the method (I) comprising the following steps (a1) to (a2) and the method (II) comprising the following steps (b1) to (b2):
{method (I)}
(a1) a step of reacting an isothiocyanate group of a compound represented by the following formula (i) with an N-terminus of a phenylalanine residue of a compound represented by the following formula (ii);
(a2) a step of reacting a linker group L^{1a} introduced into an amino group in a side chain of a lysine residue of the compound obtained in the step (a1), or the amino group, with a linker group L^{1b} or a molecular end of P of a compound represented by the following formula (iii),
{method (II)}
(b1) a step of reacting the linker group L^{1b} or the molecular end of P of the compound represented by the following formula (iii) with the linker group L^{1a} introduced into the amino group in the side chain of the lysine residue of the compound represented by the following formula (ii), or a step reacting the molecular end of P of the compound represented by the following formula (iii) with the amino group in the side chain of the lysine residue of the compound represented by the following formula (ii);
(b2) a step of reacting the amino group of the phenylalanine residue at the N-terminus of the compound obtained in the step (b1) with the isothiocyanate group of the compound represented by the following formula (i): 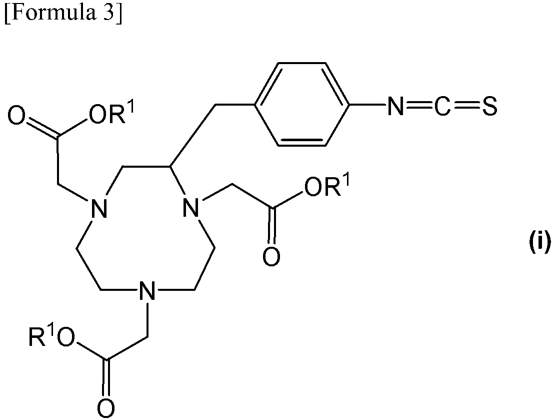
wherein R¹s are each independently a hydrogen atom or a hydrocarbon group having 1 to 8 carbon atoms, 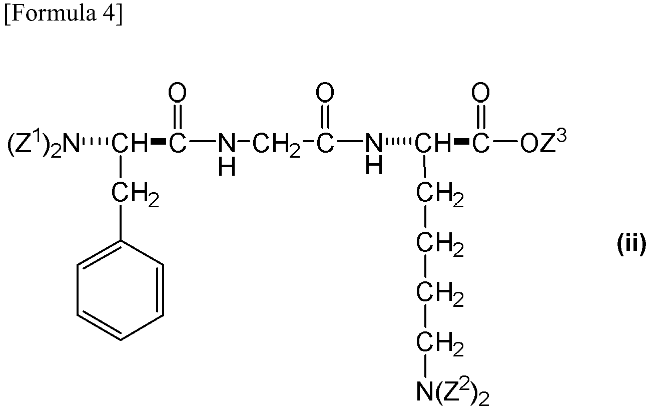
wherein Z¹ and Z² are each independently a hydrogen atom or a protecting group of an amino group, and
Z³ is a hydrogen atom, a protecting group of a carboxy group, or a bond with a solid phase,
[Formula 5]
(L^{1b})ₘ-P **(iii)**
wherein m is 0 or 1,
when m is 0, P is a protein or a peptide, and
when m is 1, L^{1b} is a linker group and P is a monovalent group derived from a protein or a peptide.

8. A method of producing the compound or a pharmaceutically acceptable salt thereof according to claim 4 or 5, comprising a step of reacting the compound or a pharmaceutically acceptable salt thereof according to claim 1 or 2 with a salt of ⁶⁷Ga, ⁶⁸Ga, ¹⁸F-Al, ⁶⁴Cu, or ⁶⁷Cu.
